# EUROPÄISCHE PATENTSCHRIFT

(11) **EP 3 229 917 B1**
(45) Veröffentlichungstag und Bekanntmachung des Hinweises auf die Patenterteilung: **21.10.2020**
(21) Anmeldenummer: 15787621.0
(22) Anmeldetag: 02.11.2015
(51) Int. Cl.: A61Q 5/10, A61K 8/22

(54) **MITTEL UND VERFAHREN ZUR BEHANDLUNG KERATINHALTIGER FASERN**
PRODUCTS AND METHOD FOR TREATING KERATIN-CONTAINING FIBERS
AGENTS ET LES MÉTHODES DE TRAITEMENT DES FIBRES CONTENANT DE LA KÉRATINE

(30) Priorität: 11.12.2014 DE 102014225556
(43) Veröffentlichungstag der Anmeldung: 18.10.2017
(73) Patentinhaber: Henkel AG & Co. KGaA, 40589 Düsseldorf (DE)
(72) Erfinder: MÜLLER, Burkhard, 40221 Düsseldorf (DE); KNAPPE, Thorsten, 22869 Schenefeld (DE)
(86) Internationale Anmeldenummer: PCT/EP2015/075375
(87) Internationale Veröffentlichungsnummer: WO 2016/091462

(56) Entgegenhaltungen:
- EP-A1- 2 468 243
- US-A- 389 670
- US-A- 3 790 031
- US-A- 3 977 826
- US-A1- 2002 074 349
- US-A1- 2002 079 377
- US-A1- 2004 065 683
- US-A1- 2013 018 333

## Beschreibung

Die Anmeldung betrifft das technische Fachgebiet der oxidativen Behandlung keratinhaltiger Fasern, insbesondere menschlicher Haare. Gegenstand der Anmeldung sind spezifische haarkosmetische Formulierungen, welche sich zur Applikation auf keratinhaltige Fasern mittels eines Entspannungsverdampfungsverfahrens eignen. Darüber hinaus sind die Verwendung dieser haarkosmetischen Formulierungen in Vorrichtungen zur Entspannungsverdampfung und Verfahren zur Farbveränderung keratinhaltiger Fasern Gegenstand der vorliegenden Anmeldung. Eine ansprechend aussehende Frisur wird heute allgemein als unverzichtbarer Teil eines gepflegten Äußeren angesehen. Um eine solche ansprechende Frisur zu realisieren werden die Haare kosmetischen Behandlungsverfahren unterworfen, die von der Reinigung mittels eines Shampoos bis zur permanenten Verformung mittels chemisch/thermischer Verfahren oder der permanenten oxidativen Farbaufhellung reichen. Zur oxidativen Farbaufhellung (Bleichen) der Haare werden wässrige Oxidationsmittelmittelzubereitungen eingesetzt. Diese Zubereitungen können vom Verbraucher beispielsweise vor Ort durch Anmischen der benötigten Komponenten hergestellt werden. Ein für die Verbrauchersicherheit wesentlicher Punkt bei dieser Art von Anwendungsverfahren ist die Vermeidung von Produktstäuben, insbesondere von Stäuben oxidationsmittelhaltiger Vorprodukte.

In der Literatur finden sich erste Ansätze zur Lösung der zuvor beschriebenen technischen Probleme. So beschreibt die deutsche Patentanmeldung DE 196 13 941 A1 ein Verfahren zur Herstellung nichtstaubender pulverförmiger Blondiermitel. Die Blondiermittel weisen mindestens eine Peroxidverbindung auf, die mit geeigneten Verdickungsmitteln versetzt und anschließend für den Transport und die Weiterverarbeitung portionsweise in wasserlösliche Beutel verpackt wird. Der Einsatz von Sprühverfahren zur Applikation oxidativer Zubereitungen ist in der Haarkosmetik wenig verbreitet.

Zu den in der Haarkosmetik genutzten Sprühsystemen zählen insbesondere die Pumpsprays oder Aerosolsprays, mit deren Hilfe die kosmetischen Zubereitungen entweder mittels mechanischer Krafteinwirkung oder mit Hilfe eines Treibmittels über ein Ventil versprüht werden. Beide Methoden haben offensichtliche Nachteile. Während Pumpsprays sich in der Regel nicht für eine lang anhaltende gleichmäßige Sprühapplikation haarkosmetischer Zubereitungen eignen, basieren Aersolsprays auf dem Einsatz von Treibmitteln oder Treibgasen, die einerseits keine kosmetische Wirkung entfalten und von denen andererseits bei unsachgemäßer Handhabung eine Gefährdung der Verbraucher ausgehen kann.

Vor diesem Hintergrund besteht ein Bedarf nach alternativen Wegen zur Zerstäubung haarkosmetischer Zubereitungen. Als ein solches alternatives Sprühverfahren hat sich die Entspannungsverdampfung erwiesen. Bei diesem Verfahren, das beispielsweise in der internationalen Patentanmeldung WO 2001/83071 A1 (Henkel) beschrieben wird, wird eine flüssige oder pastöse lösungsmittelhaltige Zusammensetzung in einem abgeschlossenen Raum auf eine Temperatur erhitzt, die über dem Siedepunkt des Lösungsmittels liegt, wodurch in der Zusammensetzung ein Überdruck erzeugt wird. Bei Entspannung (Drosselung) des Drucks verdampft die Flüssigkeit und kann nachfolgend beispielsweise mittels einer geeigneten Düse zerstäubt werden.

Auch wenn die Entspannungsverdampfung also grundsätzlich für die Sprühapplikation haarkosmetischer Zubereitungen geeignet ist, so kann gleichzeitig doch nicht jede haarkosmetische Zubereitung mittels eines Entspannungsverdampfungsverfahrens zerstäubt werden. Dies liegt einerseits an der für die Entspannungsverdampfung notwendigen Erhitzung der kosmetischen Zubereitung, andererseits an den Spezifika der durch Entspannungsverdampfung erzeugten Sprühnebel, beispielsweise der erzeugten Tröpfchengröße und Tröpfchendichte im Sprühnebel.

Aufgabe der vorliegenden Erfindung war es daher, spezifische haarkosmetische Zubereitungen zur oxidativen Behandlung keratinhaltiger Fasern zur Verfügung zu stellen, welche sich aufgrund ihrer chemischen und physikalischen Eigenschaften zur zielgerichteten Sprühapplikation mittels einer Vorrichtung zur Entspannungsverdampfung eignen. Weiterhin sollen die Zubereitungen geeignet sein, nach einer Applikation mittels eines Entspannungsverfahrens eine gute kosmetische Wirkung zu erzielen. Es hat sich gezeigt, dass zur Lösung dieser Aufgabe aus der Vielzahl der bekannten haarkosmetisch wirksamen Zubereitungen insbesondere lösungsmittelhaltige Zubereitungen mit spezifischen Gewichtsanteilen an Oxidationsmittel geeignet sind.

Ein erster Gegenstand der vorliegenden Erfindung ist somit ein kosmetisches Produkt, umfassend
a) eine kosmetische Zubereitung, enthaltend bezogen auf ihr Gesamtgewicht
   a1) 60 bis 94 Gew.-% polares Lösungsmittel;
   a2) 0,1 bis 20 Gew.-% Oxidationsmittel;
b) eine Vorrichtung zur Entspannungsverdampfung der kosmetischen Zubereitung a) mit
   b1) einem Behälter, welcher einen geschlossenen Innenraum definiert, in dem die kosmetische Zubereitung a) aufgenommen werden kann,
   b2) einem Ventil oder einem vergleichbar wirkenden Schließelement, um den wenigstens anteilsweise mit der kosmetischen Zubereitung a) befüllten Innenraum des Behälters b1) zu verschließen und zu öffnen,
   b3) einer Heizvorrichtung, um die in dem geschlossenen Innenraum des Behälters b1) befindliche kosmetische Zubereitung a) unter Druckerhöhung zu erhitzen, sowie die erhitzte kosmetische Zubereitung a) aus dem Innenraum des Behälters b1) unter Druckminderung in die Umgebung zu entspannen,
   b4) einer Düse, welche eine Zerstäubung der aus dem Behälter b1) entweichenden kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   - der Zugang zwischen Vorratsbehälter c) und Behälter b1) ein Bauteil zur Durchflussregelung aufweist, mittels dessen der Durchfluss der kosmetischen Zubereitung a) aus dem Vorratsbehälter c) in den Behälter b1) unterbrochen werden kann,
   - der Vorratsbehälter c) mindestens das Zehnfache Volumen, vorzugsweise mindestens das Zwanzigfache und insbesondere mindestens das Fünfzigfache Volumen des Behälters b1) aufweist,
   - der Druck im Inneren des Vorratsbehälters c) dem Umgebungsdruck entspricht.

Die kosmetische Zubereitung a) ist flüssig. Die kosmetische Zubereitung a) kann als Lösung oder Dispersion, beispielsweise als Emulsion oder Suspension vorliegen. Bevorzugte kosmetische Zubereitungen a) liegen in Form einer Lösung oder einer Suspension vor.

Die erfindungsgemäße kosmetische Zubereitung enthält als ersten wesentlichen Bestandteil 60 bis 94 Gew.-% eines polaren Lösungsmittels a1). Bevorzugte kosmetische Produkte sind dadurch gekennzeichnet, dass dass der Gewichtsanteil des polaren Lösungsmittels a1) am Gesamtgewicht der kosmetischen Zubereitung a) 65 bis 90 Gew.-%, vorzugsweise 70 bis 88 Gew.-% und insbesondere 75 bis 85 Gew.-% beträgt. Entsprechende Mittel zeichnen sich durch eine gute kosmetische Wirkung bei gleichzeitig guter Applizierbarkeit aus.

Zur Verbesserung der Anwendungseigenschaften erfindungsgemäßer kosmetischer Zubereitungen bei gleichzeitiger Minimierung der thermischen Belastung etwaiger Wirk- oder Hilfsstoffe im Verlauf des Entspannungsverdampfungsverfahrens, hat es sich als vorteilhaft erwiesen, polare Lösungsmittel a1) einzusetzen, welche eine Siedetemperatur (20°C, 1013 mbar) zwischen 50 und 110°C, vorzugsweise zwischen 70 und 105°C aufweisen. Als besonders geeignet haben sich dabei Ethanol und Wasser erwiesen, welche aus diesem Grund als polare Lösungsmittel a1) bevorzugt werden.

Besonders bevorzugte polare Lösungsmittel a1) oder Lösungsmittelsysteme sind
- polare Lösungsmittel a1), bei denen der Gewichtsanteil von Wasser am Gesamtgewicht des polaren Lösungsmittels a1) mehr als 80 Gew.-%, vorzugsweise mehr als 85 Gew.-% und insbesondere mehr als 90 Gew.-% beträgt.

Ein zweiter wesentlicher Bestandteil erfindungsgemäßer kosmetischer Zusammensetzungen ist das Oxidationsmittel a2). In Bezug auf die angestrebte kosmetische Wirkung der kosmetischen Zubereitung a) hat es sich als vorteilhaft erwiesen, wenn der Gewichtsanteil des Oxidationsmittels a2) am Gesamtgewicht der kosmetischen Zubereitung a) 0,5 bis 15 Gew.-%, vorzugsweise 1,0 bis 12 Gew.-% beträgt.

Ein bevorzugtes Oxidationsmittel a2) ist das Peroxidisulfat. Bevorzugte einzusetzende Peroxidisulfate sind die Alkali- und Ammoniumperoxidisulfate, vorzugsweise Natriumperoxidisulfat, Kaliumperoxidisulfat und Ammoniumperoxidisulfat, insbesondere Natriumperoxidisulfat und Kaliumperoxidisulfat, wobei Kaliumperoxidisulfat ganz besonders bevorzugt ist.

Ein ganz besonders bevorzugtes Oxidationsmittel a2) ist Wasserstoffperoxid. In einer bevorzugten Ausführungsform handelt es sich bei der kosmetischen Zubereitung a) um eine wässrige Wasserstoffperoxid-Lösung, wobei die kosmetische Zubereitung a), bezogen auf ihr Gesamtgewicht, vorzugsweise 1 bis 15 Gew.-%, besonders bevorzugt 2 bis 12 Gew.-% Wasserstoffperoxid, berechnet als 100%iges H₂O₂, enthält.

Die erfindungsgemäßen kosmetischen Produkte umfassen neben der kosmetischen Zubereitung a) weiterhin eine Vorrichtung zur Entspannungsverdampfung. Der Ausdruck "Entspannungsverdampfung" bezeichnet im Rahmen der vorliegenden Anmeldung das Entstehen von Dampf bei Absenken des Druckes in einem mit Flüssigkeit befüllten, unter Überdruck (zur Umgebung) stehenden geschlossenen Raum. Ein entsprechender Überdruck lässt sich beispielsweise erzeugen, indem eine Menge der kosmetischen Zubereitung a) in einem abgeschlossenen Raum auf eine Temperatur T₁ erhitzt wird. In dem geschlossenen Raum hat die Flüssigkeit bei gegebener Temperatur T₁ einen Druck Sättigungsdruck p₁. Wird der geschlossenen Raum beispielsweise mittels eines Ventils zu einem nicht unter Überdruck stehenden Relaxationsraum mit dem Druck p₀ < p₁ geöffnet, so sinkt der Druck in dem zuvor geschlossenen Raum ab und im Rahmen der Ausbreitung des neuen Druckniveaus verdampft die kosmetische Zubereitung a), bzw. das in der kosmetischen Zubereitung enthaltene Lösungsmittel oder Teile dieses Lösungsmittels. Der entstehende Dampf oder Sprühnebel kann für die Applikation spezifischer kosmetischer Zubereitungen genutzt werden.

Wird die kosmetische Zubereitung a) also ausgehend von Standardbedingungen (T₀ = 25°C, p₀ = 1,000 bar) in einem geschlossenen Raum erhitzt, so resultiert neben einer erhöhten Temperatur weiterhin ein erhöhter Druck der kosmetischen Zubereitung a). Dieser erhöhte Druck kann in einem Relaxationsraum auf einen Druck p₀, zum Beispiel den umgebenden Luftdruck (p₀ = 1,000 bar), entlastet werden kann, wodurch mindestens teilweise eine Verdampfung der kosmetischen Zubereitung a) erreicht wird.

Die kosmetische Zubereitung a) kann unmittelbar in dem Raum entspannt werden, in welchem sie zuvor erhitzt wurde. Die erhitzte und unter Überdruck befindliche kosmetische Zubereitung a) kann alternativ aber auch nach dem Erhitzen in einen zweiten Raum transportiert werden, in welchem dann nachfolgend die Druckentlastung erfolgt.

Bei der Entspannungsverdampfung handelt es sich mit anderen Worten um ein Verfahren, bei welchem die kosmetische Zubereitung a) in einem geschlossenen Behälter mittels einer Heizvorrichtung auf Temperaturen oberhalb der Umgebungstemperatur erhitzt wird, wobei in dem Behälter ein Druck oberhalb des Umgebungsdrucks entsteht, und die erhitzte und unter erhöhtem Druck stehende kosmetische Zubereitung a) anschließend aus dem Behälter in die Umgebung entspannt wird.

Bei einer Vorrichtung zur Entspannungsverdampfung handelt es sich demnach um eine Vorrichtung, welche einen Behälter und eine Heizvorrichtung umfasst und derart ausgestaltet ist, dass eine kosmetische Zubereitung a) in dem geschlossenen Behälter mittels der Heizvorrichtung auf Temperaturen oberhalb der Umgebungstemperatur in einer Weise erhitzt werden kann, dass in dem Behälter ein Druck oberhalb des Umgebungsdrucks entsteht und die erhitzte und unter erhöhtem Druck stehende kosmetische Zubereitung a) aus dem Behälter in die Umgebung entspannt werden kann.

Gleichzeitig mit oder nach der Druckentlastung kann die kosmetische Zubereitung a) einer Düse zugeführt werden, mittels derer beispielsweise Eigenschaften des durch die Entspannungsverdampfung erzeugten Dampfes bzw. Sprühnebels, insbesondere die Tröpfchengröße oder die Tröpfchendichte aber auch die Sprühweite und die Form des Sprühkegels beeinflusst werden können. Der Einsatz von Düsen, vorzugsweise Zerstäuberdüsen, ist daher bevorzugt. Der spezifische Düsentyp oder die spezifische Düsengestaltung wird in Abhängigkeit von den jeweiligen Sprühnebeleigenschaften gezielt festgelegt.

Der Behälter b1), in welchem die kosmetische Zubereitung erhitzt wird, ist in einer Weise ausgestaltet, die es ermöglicht, diesen Behälter während der Erhitzung der kosmetischen Zubereitung a) gegen die Umgebung vollständig abzuschließen und nach der Erhitzung, zur Ermöglichung Entspannungsverdampfung der kosmetischen Zubereitung a), zu öffnen. Dies kann beispielsweise durch ein Bauteil zur Durchflussregelung, insbesondere ein Ventil, gewährleistet werden.

Der Behälter b1), in welchem die kosmetische Zubereitung erhitzt wird, steht vorzugsweise in Kontakt mit einem weiteren Behälter, aus welchem die zur Entspannungsverdampfung vorgesehene Menge der kosmetischen Zubereitung vor der Erhitzung in den Behälter b1) überführt wird. Der Zugang zwischen diesem Vorratsbehälter und Behälter b1) ist dabei über eine entsprechende Vorrichtung, beispielsweise ein Ventil, zu öffnen und zu schließen. Dieser weitere Behälter ist vorzugsweise in Form eines Vorratsbehälters ausgestaltet, das heißt, er umfasst bevorzugt ein Vielfaches, beispielsweise mehr als das Zehnfache, vorzugsweise mehr als das Fünfzigfache der für einen Verdampfungsvorgang notwendigen Menge der kosmetischen Zubereitung. Mit anderen Worten weist der weitere Behälter/Vorratsbehälter vorzugsweise ein Vielfaches, beispielsweise mehr als das Zehnfache Volumen, vorzugsweise mehr als das Zwanzigfache und insbesondere mehr als das Fünfzigfache Volumen des Behälters b1) auf.

Der Vorratsbehälter ist kein Druckbehälter und die in dem Vorratsbehälter befindliche kosmetische Zusammensetzung befindet sich nicht unter Druck, mit anderen Worten entspricht der Druck im Inneren des Vorratsbehälters dem Umgebungsdruck (auch Luftdruck oder Atmosphärendruck). So umfassen entsprechende kosmetische Produkte beispielsweise keine Treibmittel. Auch verfügt das kosmetische Produkt über keine Pumpvorrichtung, die geeignet ist, die kosmetische Zubereitung ohne die Einwirkung der Vorrichtung zur Entspannungsverdampfung in die Umgebung freizusetzen oder zu versprühen.

Ein ganz besonders bevorzugter Gegenstand der vorliegenden Erfindung ist daher ein kosmetisches Produkt, umfassend
a) eine kosmetische Zubereitung, enthaltend bezogen auf ihr Gesamtgewicht
   a1) 60 bis 94 Gew.-% polares Lösungsmittel;
   a2) 0,1 bis 20 Gew.-% Oxidationsmittel;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht eine in dem geschlossenen Behälter b1) befindliche kosmetische Zubereitung zu erhitzen
   b3) eine Düse b3), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   ∘ der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   ∘ der Vorratsbehälter mindestens das Zehnfache Volumen, vorzugsweise mindestens das Fünfzigfache Volumen des Behälters b1) aufweist;
   ∘ der Druck im Inneren des Vorratsbehälters dem Umgebungsdruck entspricht und das kosmetische Produkt kein Treibmittel umfasst.

Bevorzugt werden weiterhin kosmetische Produkte, umfassend
a) eine kosmetische Zubereitung, enthaltend bezogen auf ihr Gesamtgewicht
   a1) 60 bis 94 Gew.-% polares Lösungsmittel;
   a2) 0,1 bis 20 Gew.-% Oxidationsmittel;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht eine in dem geschlossenen Behälter b1) befindliche kosmetische Zubereitung zu erhitzen
   b3) eine Düse b3), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   ∘ der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   ∘ der Vorratsbehälter mindestens das Zehnfache Volumen, vorzugsweise mindestens das Fünfzigfache Volumen des Behälters b1) aufweist;
   ∘ der Druck im Inneren des Vorratsbehälters dem Umgebungsdruck entspricht,
   wobei das kosmetische Produkt keine Pumpvorrichtung aufweist, die geeignet ist, die kosmetische Zubereitung a) ohne die Einwirkung der Vorrichtung zur Entspannungsverdampfung freizusetzen oder zu versprühen.

Zusammenfassend ist ein besonders bevorzugter Gegenstand der vorliegenden Erfindung daher ein kosmetisches Produkt, umfassend
a) eine kosmetische Zubereitung, enthaltend bezogen auf ihr Gesamtgewicht
   a1) 60 bis 94 Gew.-% polares Lösungsmittel;
   a2) 0,1 bis 20 Gew.-% Oxidationsmittel;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht eine in dem geschlossenen Behälter b1) befindliche kosmetische Zubereitung zu erhitzen
   b3) eine Düse b3), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   ∘ der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   ∘ der Vorratsbehälter mindestens das Zehnfache Volumen, vorzugsweise mindestens das Fünfzigfache Volumen des Behälters b1) aufweist;
   ∘ der Druck im Inneren des Vorratsbehälters dem Umgebungsdruck entspricht und das kosmetische Produkt kein Treibmittel umfasst,
wobei das kosmetische Produkt keine Pumpvorrichtung aufweist, die geeignet ist, die kosmetische Zubereitung a) ohne die Einwirkung der Vorrichtung zur Entspannungsverdampfung freizusetzen oder zu versprühen.

Neben den beiden zuvor beschriebenen Bestandteilen a1) und a2) können die erfindungsgemäßen kosmetischen Zubereitungen a) weitere Wirk- oder Hilfsstoffe enthalten, wobei insbesondere solche Wirk- oder Hilfsstoffe bevorzugt werden, welche die Herstellbarkeit, Applizierbarkeit und/oder kosmetische Wirkung erfindungsgemäßer kosmetischer Zubereitungen verbessern.

Ein erster bevorzugter Bestandteil der kosmetischen Zubereitungen a) sind die anionischen Tenside a3). Im Hinblick auf die Applizierbarkeit und kosmetische Wirkung ist es bevorzugt, wenn die kosmetische Zubereitung a), bezogen auf ihr Gesamtgewicht, 0,1 bis 6,0 Gew.-%, vorzugsweise 0,2 bis 4,0 Gew.-% und insbesondere 0,5 bis 3,0 Gew.-% anionisches Tensid a3) enthält.

Anionische Tenside im Sinne der Erfindung sind alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für solche anionischen Tenside sind, jeweils in Form der Natrium-, Kalium- und Ammonium- sowie der Mono, Di- und Trialkanolammoniumsalze mit 2 bis 4 C-Atomen in der Alkanolgruppe, lineare und verzweigte Fettsäuren mit 8 bis 30 C-Atomen (Seifen); Ethercarbonsäuren, insbesondere der Formel RO(CH₂CH₂O)ₓCH₂COOH, in der R eine lineare Alkylgruppe mit 8 bis 30 C-Atomen und x = 0 oder 1 bis 16 ist; Acylsarcoside; Acyltauride; Acylisethionate; Sulfobernsteinsäuremono- und -dialkylester sowie Sulfobernsteinsäuremono-alkylpolyoxyethylester; lineare Alkansulfonate; lineare α-Olefinsulfonate; Sulfonate ungesättigter Fettsäuren; α-Sulfofettsäuremethylester von Fettsäuren; Alkylsulfate und Alkylethersulfate, insbesondere der Formel RO(CH₂CH₂O)ₓSO₃H, in der R für eine lineare Alkylgruppe mit 8 bis 30 C-Atomen und x für 0 oder eine Zahl von 1 bis 12 steht; Gemische oberflächenaktiver Hydroxysulfonate; sulfatierte Hydroxyalkylpolyethylen- und/oder Hydroxyalkylenpropylenglykolether; Ester der Weinsäure und Zitronensäure mit Alkoholen; Alkyl- und/oder Alkenyletherphosphate der Formel RO(C₂H₄O)ₓP(=O)(OH)(OR'), worin R für einen aliphatischen, gegebenenfalls ungesättigten Kohlenwasserstoffrest mit 8 bis 30 Kohlenstoffatomen, R' für Wasserstoff, einen Rest (CH₂CH₂O)_{y}R und x und y unabhängig voneinander für eine Zahl von 1 bis 10 steht; sulfatierte Fettsäurealkylenglykolester der Formel RC(O)O(alkO)ₙSO₃H, in der R für einen linearen oder verzweigten, aliphatischen, gesättigten und/oder ungesättigten Alkylrest mit 6 bis 22 C-Atomen, alk für CH₂CH₂, CHCH₃CH₂ und/oder CH₂CHCH₃ und n für eine Zahl von 0,5 bis 5 steht; sowie Monoglyceridsulfate und Monoglyceridethersulfate. Besonders bevorzugt

Bevorzugte anionische Tenside sind Fettsäuren, Alkylsulfate, Alkylethersulfate und Ethercarbonsäuren mit 10 bis 20 C-Atomen in der Alkylgruppe und bis zu 16 Glykolethergruppen im Molekül. Besonders bevorzugt sind C₈-C₂₀-Alkylsulfate, insbesondere Natriumcetearylsulfat und Natriumlaurylsulfat, sowie C₈-C₂₀-Alkylethersulfate mit 2 bis 12, vorzugsweise 2 bis 4 Ethylenoxidgruppen, insbesondere Natriumlaurylethersulfat (INCI: Sodium Laureth Sulfate).

Eine zweite Gruppe bevorzugter Bestandteile der kosmetischen Zubereitung a) bilden die nichtionischen Tenside a4). Kosmetische Produkt, bei denen die kosmetische Zubereitung a), bezogen auf ihr Gesamtgewicht, 0,1 bis 6,0 Gew.-%, vorzugsweise 0,2 bis 4,0 Gew.-% und insbesondere 0,5 bis 3,0 Gew.-% nichtionisches Tensid a4) enthält, zeichnen sich durch gute Anwendungseigenschaften und gute kosmetische Wirkung aus.

Bevorzugte nichtionische Tenside sind PEG-Derivate von hydriertem Ricinusöl, die z. B. unter der Bezeichnung PEG Hydrogenated Castor Oil erhältlich sind, z.B. PEG-30 Hydrogenated Castor Oil, PEG-33 Hydrogenated Castor Oil, PEG-35 Hydrogenated Castor Oil, PEG-36 Hydrogenated Castor Oil, PEG-40 Hydrogenated Castor Oil oder PEG-60 Hydrogenated Castor Oil. Erfindungsgemäß besonders bevorzugt sind nichtionische Tenside ausgewählt aus der Gruppe der PEG-Derivate von hydriertem Ricinusöl, besonders bevorzugt aus der Gruppe PEG-40 Hydrogenated Castor Oil und PEG-60 Hydrogenated Castor Oil enthält.

Die Fettstoffe a5) bilden eine dritte Gruppe bevorzugter Bestandteile der kosmetischen Zubereitung a), wobei der Gewichtsanteil dieser Fettstoffe am Gesamtgewicht der kosmetischen Zubereitung a) bevorzugt 1,0 bis 25 Gew.-%, vorzugsweise 2,0 bis 22 Gew.-% und insbesondere 5,0 bis 20 Gew.-% beträgt.

Die Fettstoffe a5) können unter Normalbedingungen in fester oder flüssiger Form vorliegen. Eine erste Gruppe bevorzugter Fettstoffe a5) bilden die Wachse. Das Wachs kann natürlichen oder synthetischen Ursprungs sein. Bevorzugte Wachse schmelzen oberhalb von 40°C, besonders bevorzugt oberhalb von 50°C, insbesondere bei Temperaturen zwischen 50°C und 90°C.

Als Wachse können erfindungsgemäß feste Paraffine oder Isoparaffine, Pflanzenwachse wie Candelillawachs, Carnaubawachs, Espartograswachs, Japanwachs, Korkwachs, Zuckerrohrwachs, Ouricurywachs, Montanwachs, Sonnenblumenwachs, Fruchtwachse und tierische Wachse, wie z. B. Bienenwachse und andere Insektenwachse, Walrat, Schellackwachs, Wollwachs und Bürzelfett, weiterhin Mineralwachse, wie z. B. Ceresin und Ozokerit oder die petrochemischen Wachse, wie z. B. Petrolatum, Paraffinwachse, Microwachse aus Polyethylen oder Polypropylen und Polyethylenglycolwachse eingesetzt werden. Es kann vorteilhaft sein, hydrierte oder gehärtete Wachse einzusetzen. Weiterhin sind auch chemisch modifizierte Wachse, insbesondere die Hartwachse, z. B. Montanesterwachse, Sasolwachse und hydrierte Jojobawachse, einsetzbar.

Weiterhin geeignet sind die Triglyceride gesättigter und gegebenenfalls hydroxylierter C16-30-Fettsäuren, wie z. B. gehärtete Triglyceridfette (hydriertes Palmöl, hydriertes Kokosöl, hydriertes Rizinusöl), Glyceryltribehenat oder Glyceryltri-12-hydroxystearat, weiterhin synthetische Vollester aus Fettsäuren und Glykolen (z. B. Syncrowachs®) oder Polyolen mit 2-6 C-Atomen, Fettsäuremonoalkanolamide mit einem C12-22-Acylrest und einem C2-4-Alkanolrest, Ester aus gesättigten und/oder ungesättigten, verzweigten und/oder unverzweigten Alkancarbonsäuren einer Kettenlänge von 1 bis 80 C-Atomen und gesättigten und/oder ungesättigten, verzweigten und/oder unverzweigten Alkoholen einer Kettenlänge von 1 bis 80 C-Atomen, darunter z. B. synthetische Fettsäure-Fettalkoholester wie Stearylstearat oder Cetylpalmitat, Ester aus aromatischen Carbonsäuren, Dicarbonsäuren bzw. Hydroxycarbonsäuren (z. B. 12-Hydroxystearinsäure) und gesättigten und/oder ungesättigten, verzweigten und/oder unverzweigten Alkoholen einer Kettenlänge von 1 bis 80 C-Atomen, Lactide langkettiger Hydroxycarbonsäuren und Vollester aus Fettalkoholen und Di- und Tricarbonsäuren, z. B. Dicetylsuccinat oder Dicetyl-/stearyladipat, sowie Mischungen dieser Substanzen.

Die Wachskomponenten können auch aus der Gruppe der Ester aus gesättigten, unverzweigten Alkancarbonsäuren einer Kettenlänge von 14 bis 44 C-Atomen und gesättigten, unverzweigten Alkoholen einer Kettenlänge von 14 bis 44 C-Atomen ausgewählt werden, sofern die Wachskomponente oder die Gesamtheit der Wachskomponenten bei Raumtemperatur fest sind. Die Wachskomponenten können beispielsweise aus der Gruppe der C16-36-Alkylstearate, der C10-40-Alkylstearate, der C2-40-Alkylisostearate, der C20-40-Dialkylester von Dimersäuren, der C18-38-Alkylhydroxystearoylstearate, der C20-40-Alkylerucate ausgewählt werden, ferner sind C30-50-Alkylbienenwachs sowie Cetearylbehenat einsetzbar. Auch Silikonwachse, zum Beispiel Stearyltrimethylsilan/Stearylalkohol sind gegebenenfalls vorteilhaft. Bevorzugte Wachskomponenten sind die Ester aus gesättigten, einwertigen C20-C60-Alkoholen und gesättigten C8-C30-Monocarbonsäuren, insbesondere ein C20-C40-Alkylstearat bevorzugt, das unter dem Namen Kesterwachs® K82H von der Firma Koster Keunen Inc. erhältlich ist. Das Wachs oder die Wachskomponenten sollten bei 25 °C fest sein, jedoch im Bereich von 35-95 °C schmelzen, wobei ein Bereich von 45-85 ° C bevorzugt ist.

Weitere bevorzugte Wachskomponenten sind Fettalkohole. Als Fettalkohole können beispielsweise Stearylalkohol, Cetylalkohol, Laurylalkohol, Myristylalkohol, Arachidylalkohol, Caprylalkohol, Caprinalkohol und Behenylalkohol eingesetzt werden.

Bevorzugt ist das Wachs aus Bienenwachs (Cera Alba), Carnaubawachs, Candelillawachs, Montanwachs, Cetylpalmitat, mikrokristallinen Wachsen (mikrokristalline Paraffine) und Gemischen davon ausgewählt. Besonders bevorzugt ist der Einsatz eines Fettstoffs a5) aus der Gruppe Bienenwachs (Cera Alba), Carnaubawachs und mikrokristallinen Wachsen (mikrokristalline Paraffine).

Natürliche, chemisch modifizierte und synthetische Wachse können alleine oder in Kombination eingesetzt werden. Die erfindungsgemäße Lehre umfasst somit auch den kombinierten Einsatz von mehreren Wachsen. Weiterhin sind auch eine Reihe von Wachsmischungen, ggf. in Abmischung mit weiteren Zusätzen, im Handel erhältlich. Die unter den Bezeichnungen "Spezialwachs 7686 OE" (eine Mischung aus Cetylpalmitat, Bienenwachs, mikrokristallinem Wachs und Polyethylen mit einem Schmelzbereich von 73-75 °C; Hersteller: Kahl & Co), Polywax® GP 200 (eine Mischung von Stearylalkohol und Polyethylenglykolstearat mit einem Schmelzpunkt von 47-51 deg. C; Hersteller: Croda) und "Weichceresin® FL 400" (ein Vaseline/Vaselinöl/Wachs-Gemisch mit einem Schmelzpunkt von 50-54 deg. C; Hersteller: Parafluid Mineralölgesellschaft) sind Beispiele für erfindungsgemäß bevorzugt eingesetzte Mischungen. Eine weitere besonders bevorzugte Mischung von Wachsen a2) umfasst Bienenwachs und Carnaubawachs, gegebenenfalls in Kombination mit mikrokristallinem Wachs.

Eine zweite Gruppe erfindungsgemäß bevorzugter Fettstoffe a5) bilden die Öle. Die Öle können natürlichen oder synthetischen Ursprungs sein. Bevorzugte Öle schmelzen unterhalb von 10°C, besonders bevorzugt unterhalb von 0°C.

Bevorzugte erfindungsgemäße kosmetische Zubereitungen a) enthalten mindestens ein Öl aus der Gruppe der Silikonöle. Zur Gruppe der Silikonöle zählen insbesondere die Dimethicone, zu welchen auch die Cyclomethicone zu rechnen sind, die aminofunktionellen Silikone sowie die Dimethiconole. Die Dimethicone können sowohl linear als auch verzweigt als auch cyclisch oder cyclisch und verzweigt sein. Geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate.

Weitere bevorzugte erfindungsgemäße kosmetische Zubereitungen a) enthalten mindestens ein Öl aus der Gruppe der Esteröle, das heißt Ester von C6-C30-Fettsäuren mit C2-C30-Fettalkoholen, vorzugsweise Monoester der Fettsäuren mit Alkoholen mit 2 bis 24 C-Atomen wie beispielsweise Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkohol-caprinat/-caprylat (Cetiol® LC), n-Butylstearat, Oleylerucat (Cetioi® J 600), Isopropylpalmitat (Rilanit® IPP), Oleyl Oleate (Cetiol®), Laurinsäurehexylester (Cetioi® A), Di-n-butyladipat (Cetiol® B), Myristylmyristat (Cetiol® MM), Cetearyl Isononanoate (Cetiol® SN), Ölsäuredecylester (Cetiol® V).

Die Gruppe der Öle umfasst weiterhin die folgenden bevorzugten Substanzen:
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether.
- natürliche (pflanzliche) Öle können Triglyceride und Mischungen von Triglyceriden eingesetzt werden. Bevorzugte natürliche Öle sind Sojaöl, Rapsöl, Orangenöl, Kokosnussöl, (süßes) Mandelöl, Walnussöl, Pfirsichkernöl, Aprikosenkernöl, Avocadoöl, Teebaumöl (Tea Tree Oil), Sesamöl, Sonnenblumenöl, Tsubakiöl, Nachtkerzenöl, Reiskleieöl, Palmkernöl, Mangokernöl, Wiesenschaumkrautöl, Distelöl, Macadamianussöl, Traubenkernöl, Amaranthsamenöl, Arganöl, Bambusöl, Olivenöl, Weizenkeimöl, Kürbiskernöl, Malvenöl, Haselnussöl, Safloröl, Canolaöl, Sasanquaöl, Jojobaöl, Rambutanöl, Kakaoabutter und Shea-Butter.
- Dicarbonsäureesterwie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol® CC),
- Trifettsäureester von gesättigten und/oder ungesättigten linearen und/oder verzweigten Fettsäuren mit Glycerin,
- Polyolfettsäureester, wie beispielsweise das Handelsprodukt Hydagen® HSP (Cognis) oder Sovermol - Typen (Cognis), aus Fettsäure und/oder Fettsäurederivaten und Polyolen mit mindestens zwei Hydroxygruppen, und mit einer Kohlenstoffkette von 2 bis 30 Kohlenstoffatomen wie beispielsweise Fettsäureester von Trimethylolpropan.

Besonders bevorzugt ist der Einsatz eines Fettstoffs a5) aus der Gruppe der Silikonöle, Paraffinöle, pflanzlichen Öle und Esteröle, vorzugsweise aus der Gruppe der Silikonöle und der Paraffinöle.

Eine vierte Gruppe bevorzugter Bestandteile der kosmetischen Zubereitung a) bilden die Verdicker a6).
Bevorzugte Verdicker sind ausgewählt aus der Gruppe der polymeren organischen Verdicker. Die polymeren organischen Verdicker können vernetzt oder unvernetzt sein.

In Bezug auf die Herstellbarkeit, Applizierbarkeit und kosmetische Wirkung erfindungsgemäßer kosmetischer Zusammensetzungen hat es sich als vorteilhaft erwiesen, wenn der Gewichtsanteil des Verdickers a6) am Gesamtgewicht der kosmetischen Zubereitung a) 0,1 bis 12 Gew.-%, vorzugsweise 0,2 bis 10 Gew.-% und insbesondere 0,5 bis 8,0 Gew.-% beträgt.

Beispiele für gebräuchliche Verdicker a6) sind polymere Verdickungsmittel mit den INCI-Bezeichnungen Acrylamides Copolymer, Acrylamide/Sodium Acrylate Copolymer, Acrylamide/Sodium Acryloyldimethyltaurate Copolymer, Acrylates/Acetoacetoxyethyl Methacrylate Copolymer, Acrylic Acid/Acrylonitrogens Copolymer, Agar, Agarose, Alcaligenes Polysaccharides, Algin, Alginic Acid, Ammonium Acrylates/Acrylonitrogens Copolymer, Ammonium Acrylates Copolymer, Ammonium Acryloyldimethyltaurate/Vinyl Formamide Copolymer, Ammonium Acryloyldimethyltaurate/VP Copolymer, Ammonium Alginate, Ammonium Polyacryloyldimethyl Taurate, Amylopectin, Ascorbyl Methylsilanol Pectinate, Astragalus Gummifer Gum, Attapulgite, Avena Sativa (Oat) Kernel Flour, Bentonite, Butoxy Chitosan, Caesalpinia Spinosa Gum, Calcium Alginate, Calcium Carboxymethyl Cellulose, Calcium Carrageenan, Calcium Potassium Carbomer, Calcium Starch Octenylsuccinate, C20-40 Alkyl Stearate, Carboxybutyl Chitosan, Carboxymethyl Chitin, Carboxymethyl Chitosan, Carboxymethyl Dextran, Carboxymethyl Hydroxyethylcellulose, Carboxymethyl Hydroxypropyl Guar, Cellulose Acetate Propionate Carboxylate, Cellulose Gum, Ceratonia Siliqua Gum, Cetyl Hydroxyethylcellulose, Cholesterol/HDI/Pullulan Copolymer, Cholesteryl Hexyl Dicarbamate Pullulan, Cyamopsis Tetragonoloba (Guar) Gum, Diglycol/CHDM/lsophthalates/SIP Copolymer, Dihydrogenated Tallow Benzylmonium Hectorite, Dimethicone Crosspolymer-2, Dimethicone Propyl PG-Betaine, DMAPA Acrylates/Acrylic Acid/Acrylonitrogens Copolymer, Ethylene/Sodium Acrylate Copolymer, Gelatin, Gellan Gum, Glyceryl Alginate, Glycine Soja (Soybean) Flour, Guar Hydroxypropyltrimonium Chloride, Hectorite, Hydrated Silica, Hydrogenated Potato Starch, Hydroxybutyl Methylcellulose, Hydroxyethyl Acrylate/Sodium Acryloyldimethyl Taurate Copolymer, Hydroxyethylcellulose, Hydroxyethyl Chitosan, Hydroxyethyl Ethylcellulose, Hydroxypropylcellulose, Hydroxypropyl Chitosan, Hydroxypropyl Ethylenediamine Carbomer, Hydroxypropyl Guar, Hydroxypropyl Methylcellulose, Hydroxypropyl Methylcellulose Stearoxy Ether, Hydroxystearamide MEA, Isobutylene/Sodium Maleate Copolymer, Lithium Magnesium Silicate, Lithium Magnesium Sodium Silicate, Macrocystis Pyrifera (Kelp), Magnesium Alginate, Magnesium Aluminum Silicate, Magnesium Silicate, Magnesium Trisilicate, Methoxy PEG-22/Dodecyl Glycol Copolymer, Methylcellulose, Methyl Ethylcellulose, Methyl Hydroxyethylcellulose, Microcrystalline Cellulose, Montmorillonite, Moroccan Lava Clay, Natto Gum, Nonoxynyl Hydroxyethylcellulose, Octadecene/MA Copolymer, Pectin, PEG-800, PEG-Crosspolymer, PEG-150/Decyl Alcohol/SMDI Copolymer, PEG-175 Diisostearate, PEG-190 Distearate, PEG-15 Glyceryl Tristearate, PEG-140 Glyceryl Tristearate, PEG-240/HDI Copolymer Bis-Decyltetradeceth-20 Ether, PEG-100/IPDI Copolymer, PEG-180/Laureth-50/TMMG Copolymer, PEG-10/Lauryl Dimethicone Crosspolymer, PEG-15/Lauryl Dimethicone Crosspolymer, PEG-2M, PEG-5M, PEG-7M, PEG-9M, PEG-14M, PEG-20M, PEG-23M, PEG-25M, PEG-45M, PEG-65M, PEG-90M, PEG-115M, PEG-160M, PEG-120 Methyl Glucose Trioleate, PEG-180/Octoxynol-40/TMMG Copolymer, PEG-150 Pentaerythrityl Tetrastearate, PEG-4 Rapeseedamide, PEG-150/Stearyl Alcohol/SMDI Copolymer, Polyacrylate-3, Polyacrylic Acid, Polycyclopentadiene, Polyether-1, Polyethylene/Isopropyl Maleate/MA Copolyol, Polymethacrylic Acid, Polyquaternium-52, Polyvinyl Alcohol, Potassium Alginate, Potassium Aluminum Polyacrylate, Potassium Carbomer, Potassium Carrageenan, Potassium Polyacrylate, Potato Starch Modified, PPG-14 Laureth-60 Hexyl Dicarbamate, PPG-14 Laureth-60 Isophoryl Dicarbamate, PPG-14 Palmeth-60 Hexyl Dicarbamate, Propylene Glycol Alginate, PVP/Decene Copolymer, PVP Montmorillonite, Rhizobian Gum, Ricinoleic Acid/Adipic Acid/AEEA Copolymer, Sclerotium Gum, Sodium Acrylate/Acryloyldimethyl Taurate Copolymer, Sodium Acrylates/Acrolein Copolymer, Sodium Acrylates/Acrylonitrogens Copolymer, Sodium Acrylates Copolymer, Sodium Acrylates/Vinyl Isodecanoate Crosspolymer, Sodium Acrylate/Vinyl Alcohol Copolymer, Sodium Carbomer, Sodium Carboxymethyl Chitin, Sodium Carboxymethyl Dextran, Sodium Carboxymethyl Beta-Glucan, Sodium Carboxymethyl Starch, Sodium Carrageenan, Sodium Cellulose Sulfate, Sodium Cyclodextrin Sulfate, Sodium Hydroxypropyl Starch Phosphate, Sodium Isooctylene/MA Copolymer, Sodium Magnesium Fluorosilicate, Sodium Polyacrylate, Sodium Polyacrylate Starch, Sodium Polyacryloyldimethyl Taurate, Sodium Polymethacrylate, Sodium Polystyrene Sulfonate, Sodium Silicoaluminate, Sodium Starch Octenylsuccinate, Sodium Stearoxy PG-Hydroxyethylcellulose Sulfonate, Sodium Styrene/Acrylates Copolymer, Sodium Tauride Acrylates/Acrylic Acid/Acrylonitrogens Copolymer, Solanum Tuberosum (Potato) Starch, Starch/Acrylates/Acrylamide Copolymer, Starch Hydroxypropyltrimonium Chloride, Steareth-60 Cetyl Ether, Steareth-100/PEG-136/HDI Copolymer, Sterculia Urens Gum, Synthetic Fluorphlogopite, Tamarindus Indica Seed Gum, Tapioca Starch, TEA-Alginate, TEA-Carbomer, Triticum Vulgare (Wheat) Starch, Tromethamine Acrylates/Acrylonitrogens Copolymer, Tromethamine Magnesium Aluminum Silicate, Welan Gum, Yeast Beta-Glucan, Yeast Polysaccharides, Zea Mays (Corn) Starch.

Neben den zuvor beschriebenen Inhaltsstoffen a1), a2) und a3) bis a6) können die erfindungsgemäßen kosmetischen Mittel weitere Wirk-, Hilfs- und Pflegestoffe enthalten.

Die Zusammensetzung einiger besonders bevorzugter erfindungsgemäßer kosmetischer Zubereitungen kann den folgenden Tabellen entnommen werden (Angaben in Gew.-% bezogen auf das Gesamtgewicht des kosmetischen Mittels sofern nicht anders angegeben). Bezüglich weiterer bevorzugter Ausführungsformen dieser besonders bevorzugten Zusammensetzungen gilt mutatis mutandis das zuvor zu den erfindungsgemäßen kosmetischen Zubereitungen a) Gesagte.

| | Formel 1 | Formel 2 | Formel 3 | Formel 4 | Formel 5 |
|---|---|---|---|---|---|
| polares Lösungsmittel a1) | 65 bis 90 | 70 bis 88 | 75 bis 85 | 84 | 71 |
| Oxidationsmittel a2) | 0,1 bis 20 | 0,5 bis 15 | 1,0 bis 12 | 5,0 | 6,0 |
| optionale Additive | add 100 | add 100 | add 100 | add 100 | add 100 |

| | Formel 6 | Formel 7 | Formel 8 | Formel 9 | Formel 10 |
|---|---|---|---|---|---|
| polares Lösungsmittel a1) | 65 bis 90 | 70 bis 88 | 75 bis 85 | 84 | 71 |
| Wasserstoffperoxid a2) | 0,1 bis 20 | 0,5 bis 15 | 1,0 bis 12 | 5,0 | 6,0 |
| optionale Additive | add 100 | add 100 | add 100 | add 100 | add 100 |

| | Formel 11 | Formel 12 | Formel 13 | Formel 14 | Formel 15 |
|---|---|---|---|---|---|
| polares Lösungsmittel a1) | 65 bis 90 | 70 bis 88 | 75 bis 85 | 84 | 71 |
| Oxidationsmittel a2) | 0,1 bis 20 | 0,5 bis 15 | 1,0 bis 12 | 5,0 | 6,0 |
| anionisches Tensid a3) | 0,1 bis 6,0 | 0,2 bis 4,0 | 0,5 bis 3,0 | 2,0 | 1,0 |
| optionale Additive | add 100 | add 100 | add 100 | add 100 | add 100 |

| | Formel 16 | Formel 17 | Formel 18 | Formel 19 | Formel 20 |
|---|---|---|---|---|---|
| polares Lösungsmittel a1) | 65 bis 90 | 70 bis 88 | 75 bis 85 | 84 | 71 |
| Wasserstoffperoxid a2) | 0,1 bis 20 | 0,5 bis 15 | 1,0 bis 12 | 5,0 | 6,0 |
| anionisches Tensid a3) | 0,1 bis 6,0 | 0,2 bis 4,0 | 0,5 bis 3,0 | 2,0 | 1,0 |
| optionale Additive | add 100 | add 100 | add 100 | add 100 | add 100 |

| | Formel 21 | Formel 22 | Formel 23 | Formel 24 | Formel 25 |
|---|---|---|---|---|---|
| polares Lösungsmittel a1) | 65 bis 90 | 70 bis 88 | 75 bis 85 | 84 | 71 |
| Oxidationsmittel a2) | 0,1 bis 20 | 0,5 bis 15 | 1,0 bis 12 | 5,0 | 6,0 |
| nichtionisches Tensid a4) | 0,1 bis 6,0 | 0,2 bis 4,0 | 0,5 bis 3,0 | 0,5 | 1,5 |
| optionale Additive | add 100 | add 100 | add 100 | add 100 | add 100 |

| | Formel 26 | Formel 27 | Formel 28 | Formel 29 | Formel 30 |
|---|---|---|---|---|---|
| polares Lösungsmittel a1) | 65 bis 90 | 70 bis 88 | 75 bis 85 | 84 | 71 |
| Wasserstoffperoxid a2) | 0,1 bis 20 | 0,5 bis 15 | 1,0 bis 12 | 5,0 | 6,0 |
| nichtionisches Tensid a4) | 0,1 bis 6,0 | 0,2 bis 4,0 | 0,5 bis 3,0 | 0,5 | 1,5 |
| optionale Additive | add 100 | add 100 | add 100 | add 100 | add 100 |

| | Formel 31 | Formel 32 | Formel 33 | Formel 34 | Formel 35 |
|---|---|---|---|---|---|
| polares Lösungsmittel a1) | 65 bis 90 | 70 bis 88 | 75 bis 85 | 84 | 71 |
| Oxidationsmittel a2) | 0,1 bis 20 | 0,5 bis 15 | 1,0 bis 12 | 5,0 | 6,0 |
| Fettstoff a5) | 1,0 bis 25 | 2,0 bis 22 | 5,0 bis 20 | 2,0 | 17 |
| optionale Additive | add 100 | add 100 | add 100 | add 100 | add 100 |

| | Formel 36 | Formel 37 | Formel 38 | Formel 29 | Formel 40 |
|---|---|---|---|---|---|
| polares Lösungsmittel a1) | 65 bis 90 | 70 bis 88 | 75 bis 85 | 84 | 71 |
| Wasserstoffperoxid a2) | 0,1 bis 20 | 0,5 bis 15 | 1,0 bis 12 | 5,0 | 6,0 |
| Fettstoff a5) | 1,0 bis 25 | 2,0 bis 22 | 5,0 bis 20 | 2,0 | 17 |
| optionale Additive | add 100 | add 100 | add 100 | add 100 | add 100 |

| | Formel 41 | Formel 42 | Formel 43 | Formel 44 | Formel 45 |
|---|---|---|---|---|---|
| polares Lösungsmittel a1) | 65 bis 90 | 70 bis 88 | 75 bis 85 | 84 | 71 |
| Oxidationsmittel a2) | 0,1 bis 20 | 0,5 bis 15 | 1,0 bis 12 | 5,0 | 6,0 |
| Verdicker a6) | 0,1 bis 12 | 0,2 bis 10 | 0,5 bis 8,0 | 5,0 | 0,6 |
| optionale Additive | add 100 | add 100 | add 100 | add 100 | add 100 |

| | Formel 46 | Formel 47 | Formel 48 | Formel 49 | Formel 50 |
|---|---|---|---|---|---|
| polares Lösungsmittel a1) | 65 bis 90 | 70 bis 88 | 75 bis 85 | 84 | 71 |
| Wasserstoffperoxid a2) | 0,1 bis 20 | 0,5 bis 15 | 1,0 bis 12 | 5,0 | 6,0 |
| Verdicker a6) | 0,1 bis 12 | 0,2 bis 10 | 0,5 bis 8,0 | 5,0 | 0,6 |
| optionale Additive | add 100 | add 100 | add 100 | add 100 | add 100 |

| | Formel 51 | Formel 52 | Formel 53 | Formel 54 | Formel 55 |
|---|---|---|---|---|---|
| polares Lösungsmittel a1) | 65 bis 90 | 70 bis 88 | 75 bis 85 | 84 | 71 |
| Oxidationsmittel a2) | 0,1 bis 20 | 0,5 bis 15 | 1,0 bis 12 | 5,0 | 6,0 |
| anionisches Tensid a3) | 0,1 bis 6,0 | 0,2 bis 4,0 | 0,5 bis 3,0 | 2,0 | 1,0 |
| nichtionisches Tensid a4) | 0,1 bis 6,0 | 0,2 bis 4,0 | 0,5 bis 3,0 | 0,5 | 1,5 |
| optionale Additive | add 100 | add 100 | add 100 | add 100 | add 100 |

| | Formel 56 | Formel 57 | Formel 58 | Formel 59 | Formel 60 |
|---|---|---|---|---|---|
| polares Lösungsmittel a1) | 65 bis 90 | 70 bis 88 | 75 bis 85 | 84 | 71 |
| Wasserstoffperoxid a2) | 0,1 bis 20 | 0,5 bis 15 | 1,0 bis 12 | 5,0 | 6,0 |
| anionisches Tensid a3) | 0,1 bis 6,0 | 0,2 bis 4,0 | 0,5 bis 3,0 | 2,0 | 1,0 |
| nichtionisches Tensid a4) | 0,1 bis 6,0 | 0,2 bis 4,0 | 0,5 bis 3,0 | 0,5 | 1,5 |
| optionale Additive | add 100 | add 100 | add 100 | add 100 | add 100 |

| | Formel 61 | Formel 62 | Formel 63 | Formel 64 | Formel 65 |
|---|---|---|---|---|---|
| polares Lösungsmittel a1) | 65 bis 90 | 70 bis 88 | 75 bis 85 | 84 | 71 |
| Oxidationsmittel a2) | 0,1 bis 20 | 0,5 bis 15 | 1,0 bis 12 | 5,0 | 6,0 |
| anionisches Tensid a3) | 0,1 bis 6,0 | 0,2 bis 4,0 | 0,5 bis 3,0 | 2,0 | 1,0 |
| Fettstoff a5) | 1,0 bis 25 | 2,0 bis 22 | 5,0 bis 20 | 2,0 | 17 |
| optionale Additive | add 100 | add 100 | add 100 | add 100 | add 100 |

| | Formel 66 | Formel 67 | Formel 68 | Formel 69 | Formel 70 |
|---|---|---|---|---|---|
| polares Lösungsmittel a1) | 65 bis 90 | 70 bis 88 | 75 bis 85 | 84 | 71 |
| Wasserstoffperoxid a2) | 0,1 bis 20 | 0,5 bis 15 | 1,0 bis 12 | 5,0 | 6,0 |
| anionisches Tensid a3) | 0,1 bis 6,0 | 0,2 bis 4,0 | 0,5 bis 3,0 | 2,0 | 1,0 |
| Fettstoff a5) | 1,0 bis 25 | 2,0 bis 22 | 5,0 bis 20 | 2,0 | 17 |
| optionale Additive | add 100 | add 100 | add 100 | add 100 | add 100 |

| | Formel 71 | Formel 72 | Formel 73 | Formel 74 | Formel 75 |
|---|---|---|---|---|---|
| polares Lösungsmittel a1) | 65 bis 90 | 70 bis 88 | 75 bis 85 | 84 | 71 |
| Oxidationsmittel a2) | 0,1 bis 20 | 0,5 bis 15 | 1,0 bis 12 | 5,0 | 6,0 |
| anionisches Tensid a3) | 0,1 bis 6,0 | 0,2 bis 4,0 | 0,5 bis 3,0 | 2,0 | 1,0 |
| Verdicker a6) | 0,1 bis 12 | 0,2 bis 10 | 0,5 bis 8,0 | 5,0 | 0,6 |
| optionale Additive | add 100 | add 100 | add 100 | add 100 | add 100 |

| | Formel 76 | Formel 77 | Formel 78 | Formel 79 | Formel 80 |
|---|---|---|---|---|---|
| polares Lösungsmittel a1) | 65 bis 90 | 70 bis 88 | 75 bis 85 | 84 | 71 |
| Wasserstoffperoxid a2) | 0,1 bis 20 | 0,5 bis 15 | 1,0 bis 12 | 5,0 | 6,0 |
| anionisches Tensid a3) | 0,1 bis 6,0 | 0,2 bis 4,0 | 0,5 bis 3,0 | 2,0 | 1,0 |
| Verdicker a6) | 0,1 bis 12 | 0,2 bis 10 | 0,5 bis 8,0 | 5,0 | 0,6 |
| optionale Additive | add 100 | add 100 | add 100 | add 100 | add 100 |

Ganz besonders bevorzugte kosmetische Zubereitungen enthalten neben den zuvor beschriebenen Bestandteilen a1) bis a6) nur geringe Mengen weiterer Wirk- und Hilfsstoffe. Kosmetische Zubereitungen, dadurch gekennzeichnet, dass der Gewichtsanteil der Bestandteile a1), a2) sowie, falls vorhanden, der optionalen Bestandteilen a3) bis a6) am Gesamtgewicht der kosmetischen Zubereitung mindestens 86 Gew.-%, vorzugsweise mindestens 90 Gew.-% und insbesondere mindestens 94 Gew.-% beträgt, sind aufgrund ihrer einfachen Herstellbarkeit und guten kosmetischen Wirkung besonders bevorzugt.

Wie eingangs ausgeführt eignen sich die erfindungsgemäßen kosmetischen Zubereitungen a) in besonderer Weise für die Applikation mittels einer Vorrichtung zur Entspannungsverdampfung. Ein weiterer Gegenstand der vorliegenden Anmeldung ist daher die Verwendung einer kosmetischen Zubereitung a) enthaltend, bezogen auf ihr Gesamtgewicht,
a1) 60 bis 94 Gew.-% polares Lösungsmittel;
a2) 0,1 bis 20 Gew.-% Oxidationsmittel;
als Prozessgut in einer Vorrichtung zur Entspannungsverdampfung.

Gegenstand der vorliegenden Anmeldung ist zudem die Verwendung eines erfindungsgemäßen Produktes zur Beaufschlagung keratinhaltiger Fasern, insbesondere menschlicher Haare, mit einer kosmetischen Zubereitung a) bzw. zur Farbveränderung keratinhaltiger Fasern, insbesondere menschlicher Haare.

Ein Verfahren zur Farbveränderung keratinhaltiger Fasern, insbesondere menschlicher Haare, bei welchem die keratinhaltigen Fasern mittels einer Vorrichtung zur Entspannungsverdampfung mit einer kosmetischen Zubereitung a) enthaltend bezogen auf ihr Gesamtgewicht
a1) 60 bis 94 Gew.-% polares Lösungsmittel;
a2) 0,1 bis 20 Gew.-% Oxidationsmittel;
beaufschlagt werden, ist ein weiterer Gegenstand der vorliegenden Anmeldung. Die kosmetische Zubereitung a) wird mittels der Vorrichtung zur Entspannungsverdampfung vorzugsweise in einen Sprühnebel überführt, welcher nachfolgend die keratinhaltigen Fasern beaufschlagt.

Um eine ausreichende Sprühwirkung zu erzielen, wird die kosmetische Zubereitung a) vorzugsweise auf Temperaturen oberhalb des Siedepunktes des in der kosmetischen Zubereitung a) enthaltenen polaren Lösungsmittels oder Lösungsmittelgemisches erhitzt.

Handelt es sich bei dem polaren Lösungsmittel um Wasser oder Lösungsmittelgemische mit einem Wasseranteil oberhalb 50 Gew.-% (bezogen auf das Gesamtgewicht des Lösungsmittelgemisches), wird die kosmetische Zubereitung vorzugsweise auf Temperaturen oberhalb 100°C, vorzugsweise auf Temperaturen von 100°C und 240°C, besonders bevorzugt auf Temperaturen von 140°C bis 160°C erhitzt.

Der durch die Erhitzung der kosmetischen Zubereitung a) erzielte Überdruck beträgt in den Fällen, in denen es sich bei dem polaren Lösungsmittel um Wasser oder Lösungsmittelgemische mit einem Wasseranteil oberhalb 50 Gew.-% (bezogen auf das Gesamtgewicht des Lösungsmittelgemisches) handelt, vorzugsweise zwischen 1,1 und 8 bar, bevorzugt zwischen 1,2 und 4 bar.

Ein bevorzugter Anmeldungsgegenstand ist ein Verfahren zur Farbveränderung keratinhaltiger Fasern, insbesondere menschlicher Haare, bei welchem die keratinhaltigen Fasern mittels einer Vorrichtung zur Entspannungsverdampfung mit einer kosmetischen Zubereitung a) enthaltend bezogen auf ihr Gesamtgewicht
a1) 60 bis 94 Gew.-% polares Lösungsmittel;
a2) 0,1 bis 20 Gew.-% Oxidationsmittel;
beaufschlagt werden, wobei
- aus einem Vorratsbehälter, in dessen Inneren ein Druck herrscht, der dem Umgebungsdruck entspricht, eine Teilmenge der in diesem Vorratsbehälter befindlichen kosmetischen Zubereitung a) in einen Behälter b1) überführt wird;
- nachfolgend der Zugang zwischen Vorratsbehälter und Behälter b1) durch ein Bauteil zur Durchflussregelung, mittels dessen der Durchfluss der kosmetischen Zubereitung a) aus dem Vorratsbehälter in den Behälter b1) unterbrochen werden kann, unterbrochen wird;
- nachfolgend die in dem gegen die Umgebung abgeschlossenen Behälter b1) befindliche kosmetische Zubereitung a) mittels einer Heizvorrichtung erhitzt wird, so dass der Druck im Inneren des Behälters b1) auf Werte oberhalb des Umgebungsdrucks, vorzugsweise auf Werte zwischen 1,1 und 8 bar, insbesondere auf Werte zwischen 1,2 und 4 bar ansteigt;
- nachfolgend der unter einem Druck oberhalb des Umgebungsdrucks stehende Behälter b1) in einer Weise geöffnet, wird, welche den Austritt mindestens einer Teilmenge, vorzugsweise mindestens 50 Gew.-%, bevorzugt mindestens 80 Gew.-% und insbesondere mindestens 90 Gew.-% der in dem Behälter b1) befindlichen kosmetischen Zubereitung aus dem Behälter b1) in die Umgebung unter Minderung des zum Zeitpunkt der Behälteröffnung in dem Behälter b1) herrschenden Drucks, entspannt wird.

Die Entspannung der kosmetischen Zubereitung a) in die Umgebung erfolgt vorzugsweise unter Ausbildung eines Sprühnebels der kosmetischen Zubereitung a).

Die aus dem Behälter b1) entspannte kosmetische Zubereitung a) wird vorzugsweise auf keratinische Fasern, insbesondere menschliche Haare aufgebracht.

Verfahren, in deren Verlauf die aus dem Behälter b1) entspannte kosmetische Zubereitung vor der Beaufschlagung der keratinischen Fasern durch eine Düse geleitet wird, sind besonders bevorzugt.

## Patentansprüche

1. Kosmetisches Produkt, umfassend
a) eine kosmetische Zubereitung, enthaltend bezogen auf ihr Gesamtgewicht
a1) 60 bis 94 Gew.-% polares Lösungsmittel;
a2) 0,1 bis 20 Gew.-% Oxidationsmittel;
b) eine Vorrichtung zur Entspannungsverdampfung der kosmetischen Zubereitung a) mit
b1) einem Behälter, welcher einen geschlossenen Innenraum definiert, in dem die kosmetische Zubereitung a) aufgenommen werden kann,
b2) einem Ventil oder einem vergleichbar wirkenden Schließelement, um den wenigstens anteilsweise mit der kosmetischen Zubereitung a) befüllten Innenraum des Behälters b1) zu verschließen und zu öffnen,
b3) einer Heizvorrichtung, um die in dem geschlossenen Innenraum des Behälters b1) befindliche kosmetische Zubereitung a) unter Druckerhöhung zu erhitzen, sowie die erhitzte kosmetische Zubereitung a) aus dem Innenraum des Behälters b1) unter Druckminderung in die Umgebung zu entspannen,
b4) einer Düse, welche eine Zerstäubung der aus dem Behälter b1) entweichenden kosmetischen Zubereitung a) ermöglicht.
c) einen Vorratsbehälter für die kosmetische Zubereitung a), aus welchem die kosmetische Zubereitung a) in den Behälter b1) gelangen kann, wobei
- der Zugang zwischen Vorratsbehälter c) und Behälter b1) ein Bauteil zur Durchflussregelung aufweist, mittels dessen der Durchfluss der kosmetischen Zubereitung a) aus dem Vorratsbehälter c) in den Behälter b1) unterbrochen werden kann,
- der Vorratsbehälter c) mindestens das Zehnfache Volumen, vorzugsweise mindestens das Zwanzigfache und insbesondere mindestens das Fünfzigfache Volumen des Behälters b1) aufweist,
- der Druck im Inneren des Vorratsbehälters c) dem Umgebungsdruck entspricht.

2. Kosmetisches Produkt nach Anspruch 1, **dadurch gekennzeichnet, dass** der Gewichtsanteil des polaren Lösungsmittels a1) am Gesamtgewicht der kosmetischen Zubereitung a) 65 bis 90 Gew.-%, vorzugsweise 70 bis 88 Gew.-% und insbesondere 75 bis 85 Gew.-% beträgt.

3. Kosmetisches Produkte nach einem der vorherigen Ansprüche, **dadurch gekennzeichnet, dass** das polare Lösungsmittel a1) ausgewählt ist aus der Gruppe Wasser und Ethanol.

4. Kosmetisches Produkt nach einem der vorherigen Ansprüche, **dadurch gekennzeichnet, dass** der Gewichtsanteil des Oxidationsmittels a2) am Gesamtgewicht der kosmetischen Zubereitung a) 0,5 bis 15 Gew.-%, vorzugsweise 1,0 bis 12 Gew.-% beträgt.

5. Kosmetisches Produkt nach einem der vorherigen Ansprüche, **dadurch gekennzeichnet, dass** als Oxidationsmittel a2) Peroxidisulat, vorzugsweise Natriumperoxidisulat oder Kaliumperoxidisulfat, insbesondere Kaliumperoxidisulat eingesetzt wird.

6. Kosmetisches Produkt nach einem der Ansprüche 1 bis 4, **dadurch gekennzeichnet, dass** als Oxidationsmittel a2) Wasserstoffperoxid eingesetzt wird.

7. Verwendung einer kosmetischen Zubereitung a) enthaltend,
a) eine kosmetische Zubereitung, enthaltend bezogen auf ihr Gesamtgewicht
a1) 60 bis 94 Gew.-% polares Lösungsmittel;
a2) 0,1 bis 20 Gew.-% Oxidationsmittel;
als Prozessgut in einer b) Vorrichtung zur Entspannungsverdampfung der kosmetischen Zubereitung a) mit
b1) einem Behälter, welcher einen geschlossenen Innenraum definiert, in dem die kosmetische Zubereitung aufgenommen werden kann,
b2) einem Ventil oder einem vergleichbar wirkenden Schließelement, um den wenigstens anteilsweise mit der kosmetischen Zubereitung a) befüllten Innenraum des Behälters b1) zu verschließen und zu öffnen,
b3) einer Heizvorrichtung, um die in dem geschlossenen Innenraum des Behälters b1) befindliche kosmetische Zubereitung a) unter Druckerhöhung zu erhitzen, sowie die erhitzte kosmetische Zubereitung a) aus dem Innenraum des Behälters b1) unter Druckminderung in die Umgebung zu entspannen,
b4) einer Düse, welche eine Zerstäubung der aus dem Behälter b1) entweichenden kosmetischen Zubereitung a) ermöglicht, wobei die kosmetische Zubereitung a) in einem Vorratsbehälter c) vorhanden ist,
aus welchem die kosmetische Zubereitung a) in den Behälter b1) gelangen kann, wobei
- der Zugang zwischen Vorratsbehälter und Behälter b1) ein Bauteil zur Durchflussregelung aufweist, mittels dessen der Durchfluss der kosmetischen Zubereitung aus dem Vorratsbehälter in den Behälter b1) unterbrochen werden kann,
- der Vorratsbehälter c) mindestens das Zehnfache Volumen, vorzugsweise mindestens das Zwanzigfache und insbesondere mindestens das Fünfzigfache Volumen des Behälters b1) aufweist,
- der Druck im Inneren des Vorratsbehälters c) dem Umgebungsdruck entspricht.

8. Verwendung eines Produkts nach einem der Ansprüche 1 bis 6 zur Beaufschlagung keratinhaltiger Fasern, insbesondere menschlicher Haare, mit einer kosmetischen Zubereitung a).

9. Verwendung eines Produkts nach einem der Ansprüche 1 bis 6 zur Farbveränderung keratinhaltiger Fasern, insbesondere menschlicher Haare.

10. Verfahren zur Farbveränderung keratinhaltiger Fasern, insbesondere menschlicher Haare, bei welchem die keratinhaltigen Fasern mittels einer
b) Vorrichtung zur Entspannungsverdampfung einer kosmetischen Zubereitung a) mit
b1) einem Behälter, welcher einen geschlossenen Innenraum definiert, in dem die kosmetische Zubereitung a) aufgenommen werden kann,
b2) einem Ventil oder einem vergleichbar wirkenden Schließelement, um den wenigstens anteilsweise mit der kosmetischen Zubereitung a) befüllten Innenraum des Behälters b1) zu verschließen und zu öffnen,
b3) einer Heizvorrichtung, um die in dem geschlossenen Innenraum des Behälters b1) befindliche kosmetische Zubereitung a) unter Druckerhöhung zu erhitzen, sowie die erhitzte kosmetische Zubereitung a) aus dem Innenraum des Behälters b1) unter Druckminderung in die Umgebung zu entspannen,
b4) einer Düse, welche eine Zerstäubung der aus dem Behälter b1) entweichenden kosmetischen Zubereitung a) ermöglicht,
c) einem Vorratsbehälter für die kosmetische Zubereitung a), aus welchem die kosmetische Zubereitung a) in den Behälter b1) gelangen kann, wobei
- der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann,
- der Vorratsbehälter c) mindestens das Zehnfache Volumen, vorzugsweise mindestens das Zwanzigfache und insbesondere mindestens das Fünfzigfache Volumen des Behälters b1) aufweist,
- der Druck im Inneren des Vorratsbehälters dem Umgebungsdruck entspricht mit einer kosmetischen Zubereitung a) enthaltend bezogen auf ihr Gesamtgewicht
a1) 60 bis 94 Gew.-% polares Lösungsmittel;
a2) 0,1 bis 20 Gew.-% Oxidationsmittel;
beaufschlagt werden.

11. Verfahren nach Anspruch 10, **dadurch gekennzeichnet, dass**
- aus dem Vorratsbehälter c) eine Teilmenge der in diesem Vorratsbehälter c) befindlichen kosmetischen Zubereitung in den Behälter b1) überführt wird;
- nachfolgend der Zugang zwischen Vorratsbehälter c) und Behälter b1) durch ein Bauteil zur Durchflussregelung, mittels dessen der Durchfluss der kosmetischen Zubereitung a) aus dem Vorratsbehälter c) in den Behälter b1) unterbrochen werden kann, unterbrochen wird;
- nachfolgend die in dem gegen die Umgebung abgeschlossenen Behälter b1) befindliche kosmetische Zubereitung a) mittels einer Heizvorrichtung erhitzt wird, so dass der Druck im Inneren des Behälters b1) auf Werte oberhalb des Umgebungsdrucks, vorzugsweise auf Werte zwischen 1,1 und 8 bar, insbesondere auf Werte zwischen 1,2 und 4 bar ansteigt;
- nachfolgend der unter einem Druck oberhalb des Umgebungsdrucks stehende Behälter b1) in einer Weise geöffnet, wird, welche den Austritt mindestens einer Teilmenge, vorzugsweise mindestens 50 Gew.-%, bevorzugt mindestens 80 Gew.-% und insbesondere mindestens 90 Gew.-% der in dem Behälter b1) befindlichen kosmetischen Zubereitung a) aus dem Behälter b1) in die Umgebung unter Minderung des zum Zeitpunkt der Behälteröffnung in dem Behälter b1) herrschenden Drucks, entspannt wird.

## Claims

1. A cosmetic product, comprising
a) a cosmetic preparation, containing, based on the total weight thereof,
a1) 60 to 94 wt.% polar solvent;
a2) 0.1 to 20 wt.% oxidizing agent;
b) a device for flash evaporation of the cosmetic preparation a), comprising b1)
a container which defines a closed interior in which the cosmetic preparation a) can be received,
b2) a valve or a similarly functioning closure element for closing and opening the interior of the container b1) which is at least partly filled with the cosmetic preparation a),
b3) a heating device for heating, under increased pressure, the cosmetic preparation a) that is located in the closed interior of the container b1), and releasing, under reduced pressure, the heated cosmetic preparation a) from the interior of the container b1) into the surroundings,
b4) a nozzle which allows atomization of the cosmetic preparation a) escaping from the container b1),
c) a storage container for the cosmetic preparation a), from which storage container the cosmetic preparation a) can enter the container b1), wherein
- the access between the storage container c) and the container b1) has a component for flow control, by means of which component the flow of the cosmetic preparation a) from the storage container c) into the container b1) can be interrupted,
- the storage container c) has at least ten times the volume, preferably at least twenty times and in particular at least fifty times the volume of the container b1),
- the pressure inside the storage container c) corresponds to the ambient pressure.

2. The cosmetic product according to claim 1, **characterized in that** the proportion by weight of the polar solvent a1) with respect to the total weight of the cosmetic preparation a) is 65 to 90 wt.%, preferably 70 to 88 wt.% and in particular 75 to 85 wt.%.

3. The cosmetic product according to one of the preceding claims, **characterized in that** the polar solvent a1) is selected from the group consisting of water and ethanol.

4. The cosmetic product according to one of the preceding claims, **characterized in that** the proportion by weight of the oxidizing agent a2) with respect to the total weight of the cosmetic preparation a) is 0.5 to 15 wt.%, preferably 1.0 to 12 wt.%.

5. The cosmetic product according to one of the preceding claims, **characterized in that** peroxydisulfate, preferably sodium peroxydisulfate or potassium peroxydisulfate, in particular potassium peroxydisulfate, is used as the oxidizing agent a2).

6. The cosmetic product according to one of claims 1 to 4, **characterized in that** hydrogen peroxide is used as the oxidizing agent a2).

7. The use of a cosmetic preparation a) containing
a) a cosmetic preparation, containing, based on the total weight thereof,
a1) 60 to 94 wt.% polar solvent;
a2) 0.1 to 20 wt.% oxidizing agent;
as a process material in
b) a device for flash evaporation of the cosmetic preparation a), said device comprising
b1) a container which defines a closed interior in which the cosmetic preparation can be received,
b2) a valve or a similarly functioning closure element for closing and opening the interior of the container b1) which is at least partly filled with the cosmetic preparation a),
b3) a heating device for heating, under increased pressure, the cosmetic preparation a) that is located in the closed interior of the container b1), and releasing, under reduced pressure, the heated cosmetic preparation a) from the interior of the container b1) into the surroundings,
b4) a nozzle which allows atomization of the cosmetic preparation a) escaping from the container b1), wherein the cosmetic preparation a) is present in a storage container c) from which the cosmetic preparation a) can enter the container b1), wherein
- the access between the storage container and the container b1) has a component for flow control, by means of which component the flow of the cosmetic preparation from the storage container into the container b1) can be interrupted,
- the storage container c) has at least ten times the volume, preferably at least twenty times and in particular at least fifty times the volume of the container b1),
- the pressure inside the storage container c) corresponds to the ambient pressure.

8. The use of a product according to one of claims 1 to 6 for applying a cosmetic preparation a) to keratin fibers, in particular human hair.

9. The use of a product according to one of claims 1 to 6 for changing the color of keratin fibers, in particular human hair.

10. A method for changing the color of keratin fibers, in particular human hair, in which method, by means of
b) a device for flash evaporation of a cosmetic preparation a), said device comprising
b1) a container which defines a closed interior in which the cosmetic preparation a) can be received,
b2) a valve or a similarly functioning closure element for closing and opening the interior of the container b1) which is at least partly filled with the cosmetic preparation a),
b3) a heating device for heating, under increased pressure, the cosmetic preparation a) that is located in the closed interior of the container b1), and releasing, under reduced pressure, the heated cosmetic preparation a) from the interior of the container b1) into the surroundings,
b4) a nozzle which allows atomization of the cosmetic preparation a) escaping from the container b1),
c) a storage container for the cosmetic preparation a), from which storage container the cosmetic preparation a) can enter the container b1), wherein
- the access between the storage container and the container b1) has a component for flow control, by means of which component the flow of the cosmetic preparation a) from the storage container into the container b1) can be interrupted,
- the storage container c) has at least ten times the volume, preferably at least twenty times and in particular at least fifty times the volume of the container b1),
- the pressure inside the storage container corresponds to the ambient pressure,
a cosmetic preparation a) containing, based on the total weight thereof,
a1) 60 to 94 wt.% polar solvent;
a2) 0.1 to 20 wt.% oxidizing agent;
is applied to the keratin fibers.

11. The method according to claim 10, **characterized in that**
- some of the cosmetic preparation located in the storage container c) is transferred from said storage container c) into the container b1);
- using a component for flow control, by means of which component the flow of the cosmetic preparation a) from the storage container c) into the container b1) can be interrupted, access between the storage container c) and the container b1) is subsequently interrupted;
- the cosmetic preparation a) located in the container b1) that is sealed against the surroundings is subsequently heated by means of a heating device such that the pressure inside the container b1) increases to values above the ambient pressure, preferably to values between 1.1 and 8 bar, in particular to values between 1.2 and 4 bar;
- the container b1) which is pressurized above the ambient pressure is subsequently opened in such a manner that the discharge of at least some, preferably at least 50 wt.%, more preferably at least 80 wt.% and in particular at least 90 wt.%, of the cosmetic preparation a) located in the container b1) is released from the container b1) into the surroundings by the pressure prevailing in the container b1) at the time at which the container is opened being reduced.

## Revendications

1. Produit cosmétique, comprenant
a) une préparation cosmétique contenant, par rapport à son poids total,
a1) 60 à 94 % en poids de solvant polaire ;
a2) 0,1 à 20 % en poids d'agent oxydant ;
b) un dispositif de vaporisation éclair de la préparation cosmétique a) comportant
b1) un récipient définissant un espace intérieur fermé permettant de recevoir la préparation cosmétique a),
b2) une soupape ou un élément de fermeture à action comparable permettant de fermer et d'ouvrir l'espace intérieur du récipient b1) rempli au moins partiellement de la préparation cosmétique a),
b3) un dispositif de chauffage permettant de chauffer la préparation cosmétique a) se trouvant dans l'espace intérieur fermé du récipient b1) par augmentation de la pression, ainsi que de vaporiser la préparation cosmétique chauffée a) hors de l'espace intérieur du récipient b1) dans l'environnement par diminution de la pression,
b4) une buse permettant une atomisation de la préparation cosmétique a) s'échappant du récipient b1),
c) un récipient de stockage destiné à la préparation cosmétique a), à partir duquel la préparation cosmétique a) peut pénétrer dans le récipient b1),
- l'accès entre le récipient de stockage c) et le récipient b1) présentant un composant de régulation d'écoulement au moyen duquel l'écoulement de la préparation cosmétique a) du récipient de stockage c) vers le récipient b1) peut être interrompu,
- le récipient de stockage c) présentant au moins dix fois le volume, de préférence au moins vingt fois et en particulier au moins cinquante fois le volume du récipient b1),
- la pression à l'intérieur du récipient de stockage c) correspondant à la pression ambiante.

2. Produit cosmétique selon la revendication 1, **caractérisé en ce que** le pourcentage en poids du solvant polaire a1) est de 65 à 90 % en poids, de préférence de 70 à 88 % en poids et en particulier de 75 à 85 % en poids par rapport au poids total de la préparation cosmétique a).

3. Produit cosmétique selon l'une des revendications précédentes, **caractérisé en ce que** le solvant polaire a1) est choisi dans le groupe constitué par l'éthanol et l'eau.

4. Produit cosmétique selon l'une des revendications précédentes, **caractérisé en ce que** le pourcentage en poids de l'agent oxydant a2) est de 0,5 à 15 % en poids, de préférence de 1,0 à 12 % en poids par rapport au poids total de la préparation cosmétique a).

5. Produit cosmétique selon l'une des revendications précédentes, **caractérisé en ce que** du peroxodisulfate, de préférence du peroxodisulfate de sodium ou de potassium, en particulier du peroxodisulfate de potassium, est utilisé en tant qu'agent oxydant a2).

6. Produit cosmétique selon l'une des revendications 1 à 4, **caractérisé en ce que** du peroxyde d'hydrogène est utilisé en tant qu'agent oxydant a2).

7. Utilisation d'un produit cosmétique contenant
a) une préparation cosmétique contenant, par rapport à son poids total,
a1) 60 à 94 % en poids de solvant polaire ;
a2) 0,1 à 20 % en poids d'agent oxydant ;
en tant que matériau de processus dans un dispositif de vaporisation éclair b) de la préparation cosmétique a) comportant
b1) un récipient définissant un espace intérieur fermé permettant de recevoir la préparation cosmétique,
b2) une soupape ou un élément de fermeture à action comparable permettant de fermer et d'ouvrir l'espace intérieur du récipient b1) rempli au moins partiellement de la préparation cosmétique a),
b3) un dispositif de chauffage permettant de chauffer la préparation cosmétique a) se trouvant dans l'espace intérieur fermé du récipient b1) par augmentation de la pression, ainsi que de vaporiser la préparation cosmétique chauffée a) hors de l'espace intérieur du récipient b1) dans l'environnement par diminution de la pression,
b4) une buse permettant une atomisation de la préparation cosmétique a) s'échappant du récipient b1), la préparation cosmétique a) étant présente dans un récipient de stockage c) depuis lequel la préparation cosmétique a) peut pénétrer dans le récipient b1),
- l'accès entre le récipient de stockage et le récipient b1) présentant un composant de régulation d'écoulement, au moyen duquel l'écoulement de la préparation cosmétique du récipient de stockage vers le récipient b1) peut être interrompu,
- le récipient de stockage c) présentant au moins dix fois le volume, de préférence au moins vingt fois et en particulier au moins cinquante fois le volume du récipient b1),
- la pression à l'intérieur du récipient de stockage c) correspondant à la pression ambiante.

8. Utilisation d'un produit selon l'une des revendications 1 à 6 pour l'application d'une préparation cosmétique a) sur des fibres kératiniques, en particulier sur des cheveux humains.

9. Utilisation d'un produit selon l'une des revendications 1 à 6 pour le changement de couleur de fibres kératiniques, en particulier de cheveux humains.

10. Procédé de changement de couleur de fibres kératiniques, en particulier de cheveux humains, dans lequel une préparation cosmétique a) est appliquée sur les fibres kératiniques au moyen d'un
b) dispositif de vaporisation éclair, laquelle préparation cosmétique comporte b1)
un récipient définissant un espace intérieur fermé permettant de recevoir la préparation cosmétique a),
b2) une soupape ou un élément de fermeture à action comparable permettant de fermer et d'ouvrir l'espace intérieur du récipient b1) rempli au moins partiellement de la préparation cosmétique a),
b3) un dispositif de chauffage permettant de chauffer la préparation cosmétique a) se trouvant dans l'espace intérieur fermé du récipient b1) par augmentation de la pression, ainsi que de vaporiser la préparation cosmétique chauffée a) hors de l'espace intérieur du récipient b1) dans l'environnement par diminution de la pression,
b4) une buse permettant une atomisation de la préparation cosmétique a) s'échappant du récipient b1),
c) un récipient de stockage destiné à la préparation cosmétique a), à partir duquel la préparation cosmétique a) peut pénétrer dans le récipient b1),
- l'accès entre le récipient de stockage et le récipient b1) présentant un composant de régulation d'écoulement au moyen duquel l'écoulement de la préparation cosmétique a) du récipient de stockage vers le récipient b1) peut être interrompu,
- le récipient de stockage c) présentant au moins dix fois le volume, de préférence au moins vingt fois et en particulier au moins cinquante fois le volume du récipient b1),
- la pression à l'intérieur du récipient de stockage correspondant à la pression ambiante,
une préparation cosmétique a) contenant, par rapport à son poids total,
a1) 60 à 94 % en poids de solvant polaire ;
a2) 0,1 à 20 % en poids d'agent oxydant.

11. Procédé selon la revendication 10, **caractérisé en ce que**
- une quantité partielle de la préparation cosmétique se trouvant dans le récipient de stockage c) est transférée de ce récipient de stockage c) au récipient b1) ;
- l'accès entre le récipient de stockage c) et le récipient b1) est ensuite interrompu par un composant de régulation d'écoulement au moyen duquel l'écoulement de la préparation cosmétique a) du récipient de stockage c) vers le récipient b1) peut être interrompu ;
- la préparation cosmétique a) se trouvant dans le récipient b1) fermé à l'environnement est ensuite chauffée au moyen d'un dispositif de chauffage de sorte que la pression à l'intérieur du récipient b1) atteigne des valeurs au-dessus de la pression ambiante, de préférence des valeurs comprises entre 1,1 et 8 bar, en particulier entre 1,2 et 4 bar ;
- le récipient b1) se trouvant à une pression supérieure à la pression ambiante est ensuite ouvert de manière à vaporiser au moins une quantité partielle, de préférence au moins 50 % en poids, de façon préférée au moins 80 % en poids et en particulier au moins 90 % en poids de la préparation cosmétique a) se trouvant dans le récipient b1) hors du récipient b1) dans l'environnement, par diminution de la pression régnant dans le récipient b1) au moment de l'ouverture de celui-ci.
